# EUROPEAN PATENT SPECIFICATION

(11) **EP 3 600 489 B1**
(45) Date of publication and mention of the grant of the patent: **28.09.2022**
(21) Application number: 18718032.8
(22) Date of filing: 23.03.2018
(51) Int. Cl.: A61M 3/02

(54) **BODY CAVITY IRRIGATION INTEGRATED MANUAL CONTROLLER AND PUMP DEVICE**
INTEGRIERTE MANUELLE STEUERUNG UND PUMPENVORRICHTUNG FÜR KÖRPERHOHLRAUMSPÜLUNG
DISPOSITIF DE COMMANDE MANUELLE ET DISPOSITIF DE POMPE INTÉGRÉS POUR L'IRRIGATION D'UNE CAVITÉ CORPORELLE

(30) Priority: 29.03.2017 US 201762478279 P
(43) Date of publication of application: 05.02.2020
(73) Proprietor: Hollister Incorporated, Libertyville, IL 60048 (US)
(72) Inventor: HENRY, Jerome, A., Libertyville, IL 60048 (US); ARNOLD, William, K., Libertyville, IL 60048 (US); MATESI, Donald, V., Libertyville, IL 60048 (US); GAMBLIN, Denise, Libertyville, IL 60048 (US); GLENNON, Mary, L., Libertyville, IL 60048 (US); MCCARRICK, Finn, Libertyville, IL 60048 (US); CULLUM, Malford, E., Libertyville, IL 60048 (US)
(74) Representative: FRKelly
(86) International application number: PCT/US2018/024174
(87) International publication number: WO 2018/183128

(56) References cited:
- WO-A1-2004/006993
- WO-A1-2016/007536
- WO-A2-2010/115431
- US-B2- 8 579 850

## Description

### CROSS-REFERENCE TO RELATED APPLICATIONS

This application claims the benefit of and priority to U.S. Provisional Patent Application No. 62/478,279, filed March 29, 2017.

### FIELD OF THE INVENTION

The present disclosure relates generally to body cavity irrigation devices, methods and systems and, in particular, to a body cavity irrigation integrated manual controller and pump device.

### BACKGROUND

Transanal irrigation (TAI) is a process used by individuals who have bowel management issues, such as incontinence, constipation or other neurogenic bowel dysfunction (NBD). Alternatively, TAI may be used for regular bowel evacuations by individuals who are incapacitated due to illness or other medical conditions or injuries (such as spinal cord injury) and thus lack the mobility to access a toilet. During TAI, water or other lavage liquid is introduced into the rectum and colon through a device positioned through the anus so that feces are flushed and evacuated. This creates pseudo-continence for the patient/user. Furthermore, individuals that are bedridden may develop fecal impaction. Such bowel obstructions may be removed via TAI.

Systems for performing TAI currently on the market allow the user to introduce water into the bowel through a rectal catheter while the user sits on a toilet or a commode/shower chair or lays in a bed. The user introduces an amount of water or other liquid into the bowel (typically 500-700 mL) in order to flush out stool located in the bowel passage. The user typically introduces the water, waits for a period of time and then allows gravity to flush the water and stool out of the body. The rectal catheter may have an inflatable/deflatable balloon to assist in retention of the catheter during water introduction. The balloon is typically inflated by a fluid such as air or water.

For TAI users, independence, dexterity, and ease of use are important needs that must be addressed by a TAI system or method.

A prior art TAI device is shown in U.S. Patent No. 8,579,850 to Bjerregaard and uses water to inflate the balloon of a rectal catheter. This system has single-lumen tubing that provides water from a reservoir to a controller. The system features dual-lumen tubing from the controller to the catheter. One of these dual lumens enables the rectal catheter balloon to be inflated with water from the reservoir and later deflated; while the second lumen accommodates water transfer from the reservoir into the rectum. When the catheter balloon is deflated, a liquid communication channel is created so that water returning from the deflated balloon travels via the controller into the lumen towards the catheter, and thus into the rectum. As a result, the water from the deflated balloon does not return to the water reservoir. A disadvantage of such a system is that water from the balloon is unnecessarily directed into the patient.

Furthermore, prior art manual pump TAI systems, such as the systems of the Bjerregaard '850 patent and U.S. Patent No. 8,657,801 to Nielsen et al., use a squeeze bulb that is separate from the controller to pump fluids wherein the user/patient must hold the squeeze bulb in addition to squeezing it to activate the pumping action. This can be awkward for a patient/user and may result in inadequate pumping pressure.

Other devices for irrigating a body cavity using irrigation comprising a pump mechanism and a valve mechanism are disclosed in WO2004/006993 (A1) and WO2010/115431 (A2).

Accordingly is a desire to develop a TAI controller and pump device, system and/or method for bowel management that addresses at least some of the above issues.

### SUMMARY

The invention is defined by the features of independent claim 1. There are several aspects of the present subject matter which may be embodied separately or together in the devices and systems described and claimed below. These aspects may be employed alone or in combination with other aspects of the subject matter described herein, and the description of these aspects together is not intended to preclude the use of these aspects separately or the claiming of such aspects separately or in different combinations as set forth in the claims appended hereto.

In one aspect, a device for irrigating a body cavity using irrigation liquid from a reservoir and a catheter having a flushing port and a retention balloon includes a device housing, a pump mechanism positioned within the device housing and a valve mechanism positioned within the device housing. The valve mechanism includes a valve housing having an inlet port that is in fluid communication with the pump mechanism and configured to be placed in fluid communication with the reservoir. The valve housing also includes a plurality of outlet ports. At least one of the plurality of outlet ports is configured to be placed in fluid communication with the flushing port of the catheter and at least one of the plurality of outlet ports is configured to be placed in fluid communication with the retention balloon of the catheter. A valve member is rotatably positioned within the valve housing. The valve member includes a valve aperture in fluid communication with the inlet port that may be selectively placed in fluid communication with each of the plurality of outlet ports.

### BRIEF DESCRIPTION OF THE DRAWINGS

Fig. 1 is a perspective view of an embodiment of an integrated controller and pump device with the pumping handle in the use position;
Fig. 2 is a side elevational view of the device of Fig. 1;
Fig. 3 is a perspective view of the device of Figs. 1 and 2 with the pumping handle in the storage position;
Fig. 4 is an enlarged perspective view of the pumping handle, pump mechanism and valve mechanism of the device of Figs. 1-3;
Fig. 5 is a cross-sectional view of the pump and valve mechanisms of Fig. 4;
Fig. 6 is an exploded view of the pump and valve mechanisms of Fig. 5;
Fig. 7 is an enlarged perspective view showing the bottom of the pump and valve housing of Figs. 5-6;
Fig. 8 is a schematic of a body cavity irrigation system including the integrated controller and pump device of Figs. 1-7;
Fig. 9A is an enlarged top perspective view of the valve mechanism of the device of Figs. 1-7 configured to prime the system of Fig. 8;
Fig. 9B is an enlarged top perspective view of the valve mechanism of the device of Figs. 1-7 configured to inflate the retention balloon of the catheter of the system of Fig. 8;
Fig. 9C is an enlarged top perspective view of the valve mechanism of the device of Figs. 1-7 configured for flushing through the catheter of the system of Fig. 8;
Fig. 9D is an enlarged top perspective view of the valve mechanism of the device of Figs. 1-7 configured to deflate the retention balloon of the system of Fig. 8;
Fig. 10A is a simplified schematic view of the pump mechanism of the device of Figs. 1-7 during an intake stroke;
Fig. 10B is a simplified schematic view of the pump mechanism of the device of Figs. 1-7 at top dead center of the stroke;
Fig. 10C is a simplified schematic view of the pump mechanism of the device of Figs. 1-7 during a pumping stroke;
Fig. 11 is a perspective view of an alternative embodiment of an integrated controller and pump device that uses a piston pump mechanism;
Fig. 12 is a perspective view of another alternative embodiment of an integrated controller and pump device that uses a piston pump mechanism;
Fig 13 is a perspective view of another alternative embodiment of an integrated controller and pump device that uses a piston pump mechanism;
Fig. 14 is a perspective view of an integrated controller and pump device that uses an integrated bulb pump mechanism;
Figs. 15A, 15B and 15C are perspective, top plan and side elevational views, respectively, of an integrated controller and pump device that uses an integrated dial and bulb pumping mechanism;
Fig. 16 is a perspective view of an integrated controller and pump device that uses a rotary pump mechanism;
Fig. 17 is a side elevational view of the device of Fig. 16;
Fig. 18 is a schematic view of an embodiment of the pump mechanism of the device of Figs. 16 and 17;
Fig. 19 is a perspective exploded view of the ratchet wheel drive mechanism for the pump mechanism of Fig. 18;
Fig. 20 is a schematic of a body cavity irrigation system including the integrated controller and pump device of Figs. 16 and 17;
Fig. 21 is a schematic view of an alternative embodiment of the pump mechanism of the device of Figs. 16 and 17 and the system of Fig 20;
Fig. 22 is a perspective view of an alternative embodiment of an integrated controller and pump device that uses a rotary pump mechanism;
Fig. 23 is perspective view of an alternative embodiment of an integrated controller and pump device; and
Fig. 24 is a perspective view of an integrated controller and pump device that uses a bellows as the pump mechanism.

### DETAILED DESCRIPTION OF EMBODIMENTS

While the embodiments are described below in terms of use in a transanal irrigation procedure, it is to be understood that they could instead be used to irrigate other body cavities of a user including, but not limited to, stomas and body cavities accessible by stomas.

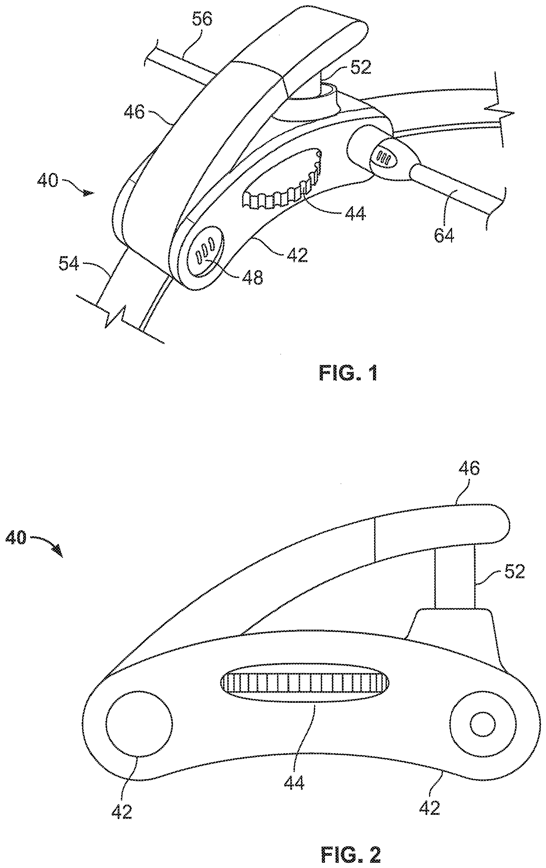

An integrated controller and pump device is indicated in general at 40 in Figs. 1-3. The device includes a housing 42 within which a rotating valve selector wheel 44 is positioned. In addition, a pumping handle or lever 46 is pivotally attached at its proximal end by pin 48. As best shown in Fig. 2, the distal end of the pumping handle 46 is attached to the top end of a piston rod 52. As will be described in greater detail below, the bottom end of the piston rod 52 is connected to a piston positioned in a pumping chamber.

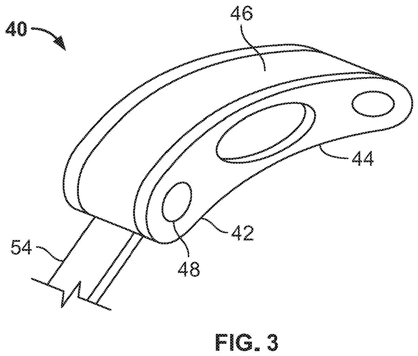

As illustrated in Fig. 3, the pumping handle 46 may be provided with a latching arrangement so that it folds against or nests with the device housing 42 to provide a compact storage and transport profile for the device. The configuration of Fig. 3 also reduces the chance of accidental actuation of the pumping handle 46.

A strap 54 (Figs. 1 and 2) is attached to ends of the housing 42, and the housing preferably features an arcuate shape so that the device may be conveniently mounted on the leg of a user.

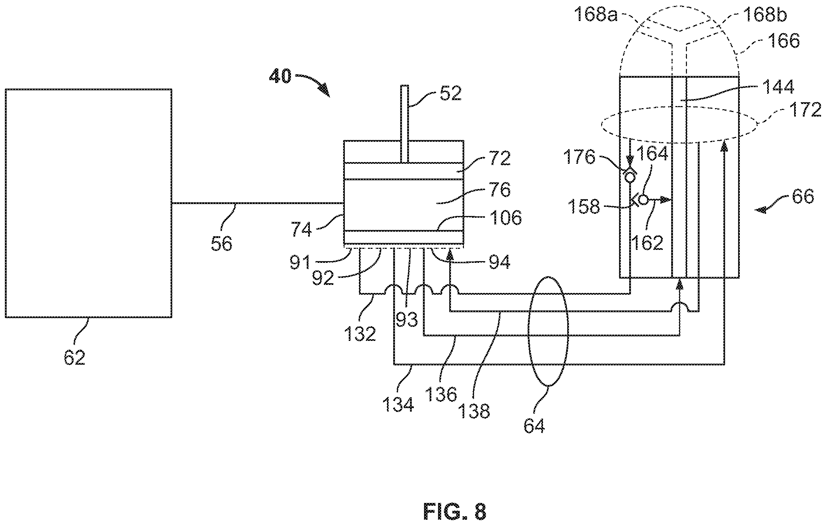

With reference to Fig. 1, inlet tubing 56 connects the pumping mechanism of the device 40 to an irrigant or lavage liquid reservoir (illustrated at 62 in Fig. 8). In addition, outlet tubing 64, which may be composed of multiple conduits, connects the pumping mechanism of the device 40 to a rectal catheter, indicated in general at 66 in Fig. 8. Alternatively, multiple outlet tubings could connect the pumping mechanism to the recited catheter.

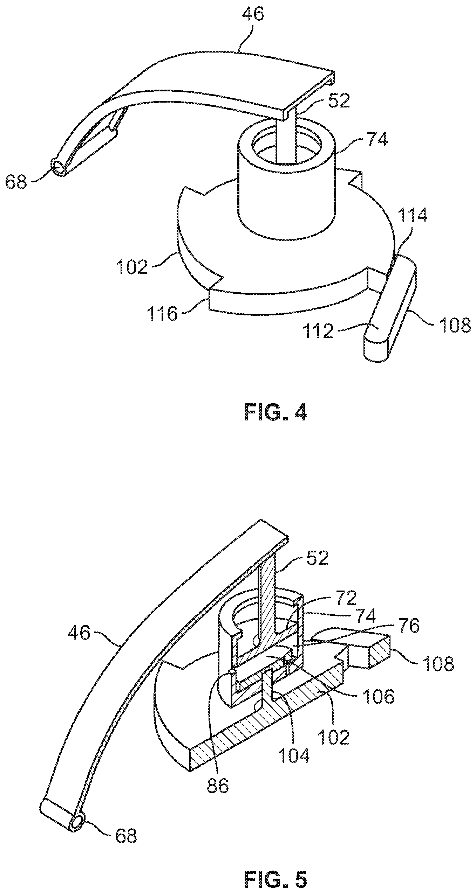

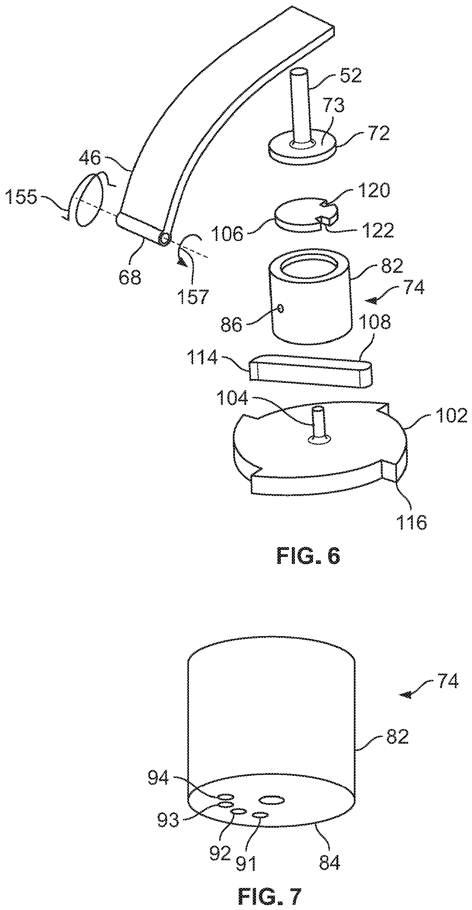

With reference to Figs. 4-6, the proximal end of the pumping handle 46 is provided with a cylindrical portion 68 which receives pin 48 of Figs. 1-3 to pivotally mount the pumping handle 46 to the device housing. As noted previously, a piston rod 52 is attached by its top end to the distal end of the pumping handle 46. The bottom end of the piston rod 52 is attached to a piston, indicated at 72 in Figs. 5 and 6. The piston rod 52 and piston 72 may be independently formed and joined together, or the two components may be integrally formed as a single piece, such as from molded plastic.

With reference to Fig. 6, the piston 72 is provided with an inlet valve 73, which includes an opening or passage formed through the piston. A valve member (not shown) (e.g., tab, flap or door) is positioned on the underside of the piston and, as explained in greater detail below, covers the passage during downward travel of the piston, but permits liquid to travel through the passage during upward movement of the piston.

As illustrated in Figs. 4 and 5, a cup-shaped pump and valve housing 74 receives the piston 72 in a sliding fashion so that a pumping chamber 76 (Fig. 5) is formed beneath the piston 72. With reference to Figs. 6 and 7, the pump and valve housing includes a cylindrical sidewall 82 and a disc-shaped bottom 84. The top of the housing 74 is open to permit passage of the piston rod 52. The sidewall 82 is provided with an inlet port 86 (Figs. 5 and 6) that communicates with the pumping chamber 76. With reference to Fig. 7, the bottom 84 of the pump and valve housing is provided with outlet ports 91, 92, 93 and 94.

With reference to Figs. 4-6, a ratchet wheel 102 is mounted below the pump and valve housing 74 within the device housing (42 of Figs. 1-3) and features a central, upwardly-extending shaft 104. A valve disc 106 (Figs. 5 and 6) is secured to the top end of the shaft 104 within the pumping chamber of the pump and valve housing so that the valve disc 106 rotates with the ratchet wheel 102 about an axis passing longitudinally through shaft 104 when the ratchet wheel is turned.

A pawl 108 is pivotally mounted within the device housing (42 in Figs. 1-3), such as via a pin 112 (Fig. 4) at the proximal end. The distal end of the pawl is provided with a tapered tip 114. The pawl is provided with a spring (not shown) that urges the pawl tip 114 towards and into engagement with teeth 116 formed on the circumference of the ratchet wheel.

The valve selector wheel 44 (Figs. 1-3) of the device is connected to the ratchet wheel 102 via a gear train, belt drive, or any other coupling arrangement known in the art, so that the ratchet wheel 102 of Figs. 4-6, and thus the valve disk 106 of Figs. 5 and 6, turns when the valve selector wheel 44 is turned. The pawl 108 and profile of the teeth 116 of the ratchet wheel 102 permits the ratchet wheel, and thus the valve selector wheel 44 and valve disk 106, to be rotated in only a single direction and holds the components in a selected position.

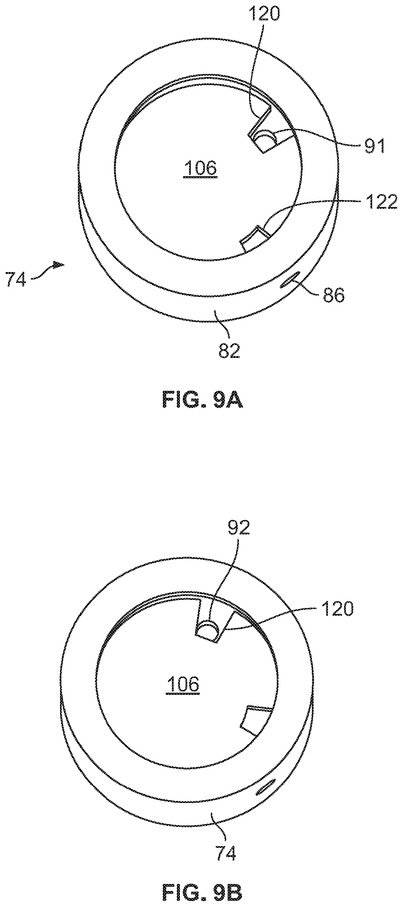

As illustrated in Figs. 6 and 9A-9D, the valve disk 106 is provided with valve apertures 120 and 122. While notches are illustrated for the valve apertures, the apertures may be any other type of openings formed in the disk such as holes. Rotating the valve disk 106, via manipulation of the valve selector wheel (44 of Figs. 1-3) of the device, selectively places the valve apertures over the outlet ports 91, 92, 93 and 94 so that the integrated controller and pump device may be configured for performing different steps or stages in a transanal irrigation procedure.

When the user rotates the valve selector wheel of the device into the Prime position (which may be indicated, for example, as position "1" on the wheel), as illustrated in Fig. 9A, the disk 106 rotates and valve aperture 120 is positioned over outlet port 91, the device is configured to prime the flushing passage of the catheter. The remaining outlet ports (92-94 of Fig. 8) are covered by the disk 106. With reference to Fig. 8, the outlet tubing 64 includes conduits 132, 134, 136 and 138. Conduit 132 is connected to outlet port 91 and, as explained in greater detail below, directs the flushing liquid to the flushing passage 144 of the catheter 66.

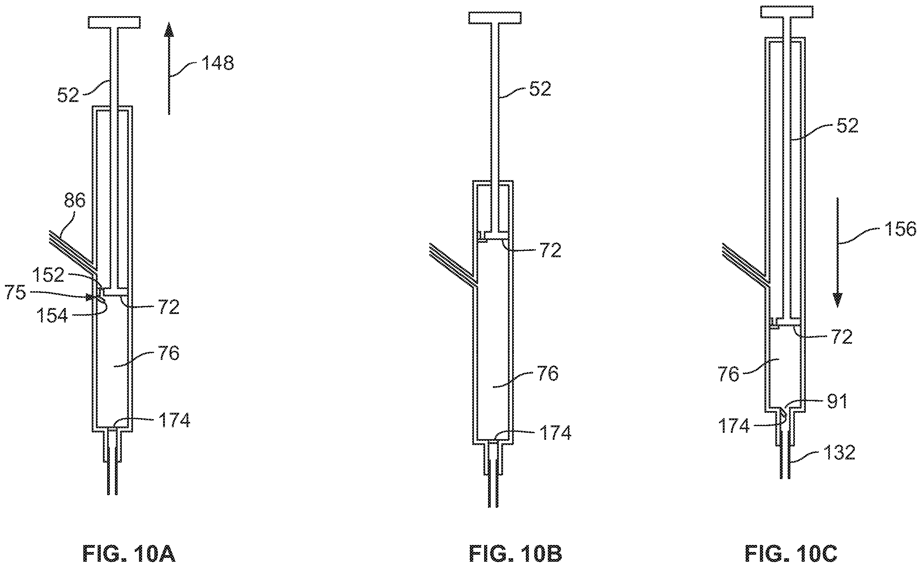

Continuing with Fig. 8, water, or any other liquid irrigant, is next pumped by the user from reservoir 62 to the catheter flushing passage 144 via actuation of the pumping handle (46 of Figs. 1-6) of the integrated controller and pump device. More specifically, with reference to Fig. 10A, as the pumping handle (46 of Figs. 1-6) is pivoted counter-clockwise or upwards, the piston rod 52, and thus piston 72, move upwards, as illustrated by arrow 148. As piston 72 moves upwards, liquid is drawn into the pumping chamber 76 through the opening 152 and past the valve member 154 of the inlet valve 73 of the piston.

The pumping handle 46 of Figs. 1-6 may be provided with a spring, such as torsion spring 155 of Fig. 6, positioned between the proximal end of the handle 46 and the device housing 42, to urge the handle in the direction of arrow 157 of Fig. 6 and towards a position corresponding to the piston 72 being in the top dead center position.

Next, with reference to Fig. 10B, when the piston 72 reaches the top dead center position, the valve member 154 of the piston inlet valve closes and the pumping chamber 76 is filled with liquid.

When the pump handle (46 of Figs. 1-6) is moved downwards or clockwise, the piston 72 moves downward, as illustrated by arrow 156 of Fig. 10C, while the valve member 154 of the piston inlet valve remains closed. As a result, the liquid within the cylinder is forced or pushed out of the pumping chamber 76 through the outlet port 91 and conduit 132.

With reference to Fig. 8, the liquid traveling through conduit 132 is directed to the catheter 66 where it encounters junction 158. Branch 162 features a priming check valve 164 that permits the liquid to flow to the catheter flushing passage 144. The catheter flushing passage 144 leads to the catheter tip, illustrated in phantom 166, which includes eyelets 168a and 168b. The user knows that the catheter has been primed when he or she observes the flushing liquid exiting the eyelets 168a and 168b.

The configuration of the catheter may vary from what is illustrated, and it may contain a different number of eyelets.

After the unit is primed, the user will insert the catheter into their rectum, per clinical instructions. The user next rotates the valve selector wheel 44 (Figs. 1-3) into the Retention Balloon Inflation position (which may be indicated, for example, as position "2" on the wheel). This causes the disk 106 to rotate into the position illustrated in Fig. 9B, where outlet port 92 is aligned with valve aperture 120. The remaining outlet ports are covered by the disk.

As illustrated in Fig. 8, outlet port 92 communicates with conduit 134, which leads to the catheter retention balloon 172. The user then actuates the pumping handle 46 (Figs. 1-6) which causes liquid to be pumped, in the manner described above with respect to Figs. 10A-10C, to the retention balloon 172 so as to inflate it. Water exclusively enters the retention balloon each time the user compresses the pumping handle.

As illustrated in Figs. 10A-10C, outlet ports 92 and 93 are each provided with an outlet valve member 174 which is closed during upward movement of the pumping piston 72 (Fig. 10A) and opens during downward movement of the pumping piston. The outlet valve is absent from the outlet ports 91 and 94 to permit liquid to enter the pumping chamber 76 during deflation of the retention balloon, as described below.

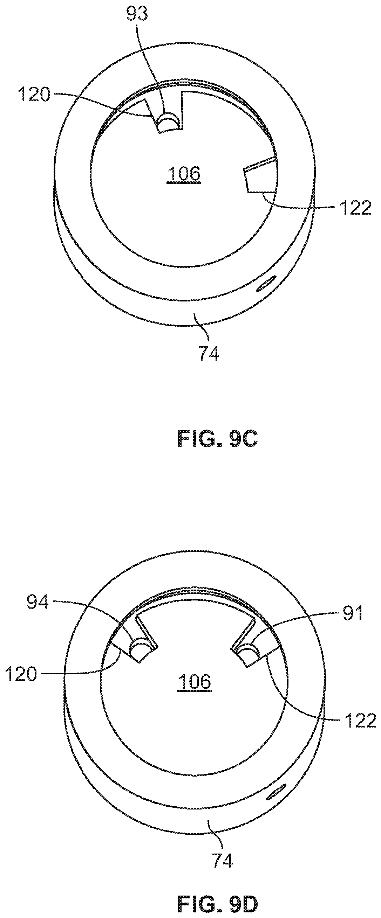

The next step in the TAI process is to deliver water into the distal rectum. The user rotates the valve selector wheel 44 (Figs. 1-3) to the Flushing position (which may be indicated, for example, as position "3" on the wheel), which in turn, as illustrated in Fig. 9C, rotates the disk 106 to reveal the outlet port 93 through valve aperture 120 (the remaining outlet ports closed by the disk 106). As illustrated in Fig. 8, outlet port 93 communicates with conduit 136, which leads to the flushing passage 144 of the catheter 66. The user again manipulates the pumping handle (46 of Figs. 1-6) of the device to deliver flushing liquid into the rectum of the user through the flushing passage 144 (Fig. 8) and eyelets 168a and 168b of the catheter 66.

Conduit 136 preferably features a diameter that is larger than the diameter of conduit 134 so that appropriate amounts of liquid are delivered to the rectum and retention balloon during actuation of the pumping handle of the device.

After the correct volume of water has been delivered into the rectum, the user must deflate the retention balloon so that they can remove the catheter from their rectum. Hence, the user rotates the dial to the Retention Balloon Deflation position (which may be indicated, for example, as position "4" on the wheel). This causes the disk 106 to rotate into the position illustrated in Fig. 9D which results in valve apertures 120 and 122 being aligned with outlet ports 94 and 91, respectively. As a result, with reference to Fig. 8, the water inside the retention balloon 172 is forced by the resiliency of the balloon to return to the water reservoir 62 via the conduit 138, pumping chamber 76 and tubing 56, thus enabling the balloon to deflate.

A balloon deflation check valve 176 (Fig. 8) also permits liquid from the retention balloon 172 to travel through conduit 132 and port 91 to expedite deflation of the balloon. This feature is optional and the omission of the feature would permit the balloon deflation check valve 166, and the associated branch between the balloon and the junction 158, to be eliminated so that liquid from the deflating balloon only travels through conduit 138. In such an embodiment, only outlet port 94 would be uncovered in Fig. 9D.

It should be noted from the above that the lumens of conduits 132, 134, 136 and 138 are never placed in communication with each other during a TAI procedure, i.e. there is no fluid communication between the lumens.

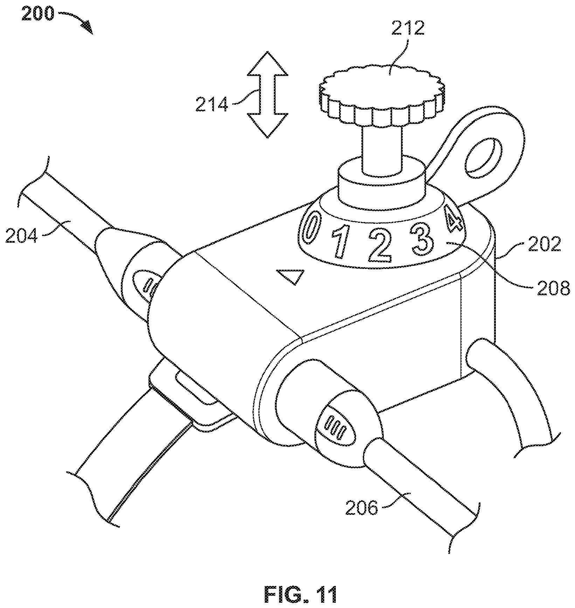

An alternative embodiment of an integrated controller and pump device is indicated in general at 200 in Fig. 11. As with the embodiment described above, the device 200 includes a housing 202, inlet tubing 204 and outlet tubing 206 (containing multiple conduits or multiple tubings). The device 200 also includes a valve selector wheel 208 that permits device configurations (Prime, Retention Balloon Inflation, Flushing, Retention Balloon Deflation) to be selected. The device differs from the embodiment of Figs. 1-6 in that a pumping knob 212 is substituted for the pumping handle 46 of Figs. 1-6. The pumping knob is articulated in the manner indicated by arrows 214 to pump liquids to the catheter. Otherwise, the functionality of the valve and pump mechanisms of the device 200 are the same as described above with reference to Figs. 8-10C.

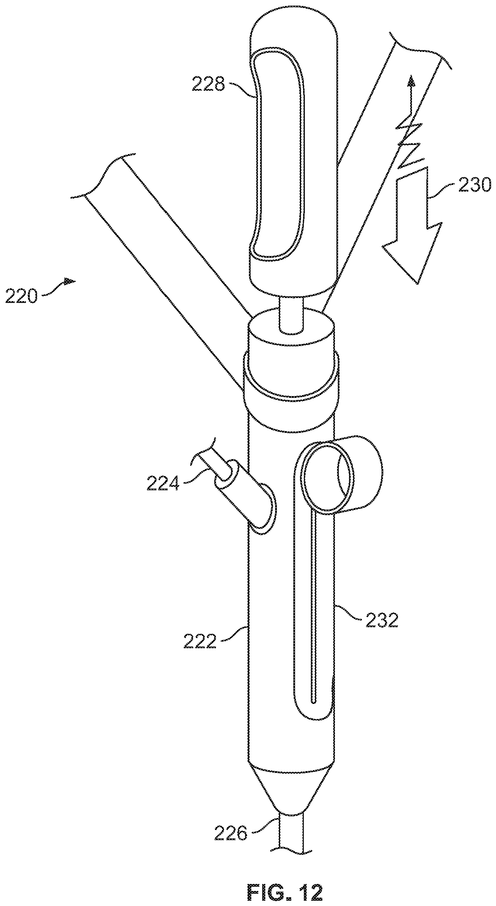

Another alternative embodiment of an integrated controller and pump device is indicated in general at 220 in Fig. 12. In this embodiment, the housing 222 is elongated and is provided with inlet and outlet tubing 224 and 226. The housing 222 contains valve and pump mechanisms as described above with reference to Figs. 8-10C. A pumping handle 228 replaces the pumping handle 46 of Figs. 1-6 and the pumping knob 212 of Fig. 11. Pumping handle 228 is actuated as indicated by arrows 230 to pump liquid through the device. Preferably, a spring urges the pumping handle 228 into a position corresponding to the top dead center position for the piston of the pumping mechanism (illustrated in Fig. 10B).

Furthermore, instead of the valve selector wheel 44 of Figs. 1-3 or 208 of Fig. 11, the device 220 of Fig. 12 is provided with a sliding valve selector switch 232. The device housing 22 contains a linkage so that upward and downward movement of the switch 232 along the housing surface results in rotation of the ratchet wheel 102 of Figs. 4-6. As described previously, this causes the valve disk 106 of Figs. 9A-9D to rotate so that the device configurations (Prime, Retention Balloon Inflation, Flushing, Retention Balloon Deflation) may be selected. The linkage between the sliding switch 232 and ratchet wheel (102 of Figs. 4-6) may include, as an example only, a bevel gear and/or rack and pinion gear arrangement arrangement. As another example, rotation of the ratchet wheel may be spring-biased and a line (such as string) may wrap around a portion of the ratchet wheel so that pulling the line linearly (via a pulley and switch 232) rotates the ratchet wheel.

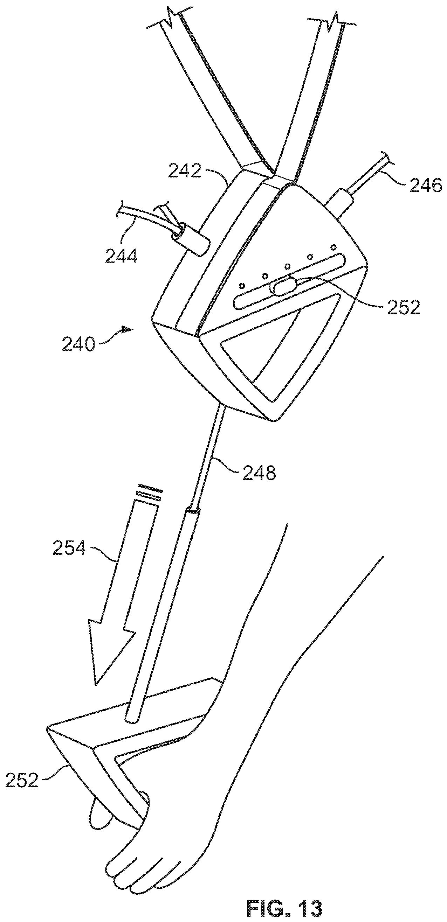

Another alternative embodiment of an integrated controller and pump is indicated in general at 240 in Fig. 13. As in previous embodiments, the housing 242 is is provided with inlet and outlet tubing 244 and 246. The housing 242 contains valve and pump mechanisms as described above with reference to Figs. 8-10C with a cord 248 attached by one end to the piston rod (52 of Fig. 8). A spring urges the piston rod into a position corresponding to either the top dead center position for the piston of the pumping mechanism (illustrated in Fig. 10B) or the bottom-most position for the piston.

A handle 252 (Fig. 13) is attached to the other end of the cord 248. When handle 252 is moved as indicated by arrow 254 in Fig. 13, the cord 248 extends out of the housing 242 and the piston of the pump mechanism is moved against the urging of the spring to either draw liquid into the pumping chamber or to push it out of the pumping chamber. The user then permits the cord 248 to be retracted back into the device housing 242 (under the urging of the spring) to repeat the stroke and thus pump liquid through the device.

Like the device 220 of Fig. 12, the device 240 of Fig. 13 is provided with a sliding valve selector switch 252. The device housing 242 contains a linkage so that movement of the switch 252 results in rotation of the ratchet wheel 102 of Figs. 4-6. As described previously, this causes the valve disk 106 of Figs. 9A-9D to rotate so that the device configurations (Prime, Retention Balloon Inflation, Flushing, Retention Balloon Deflation) may be selected. The linkage between the sliding switch 252 and ratchet wheel (102 of Figs. 4-6) may include, as an example only, a rack and pinion gear arrangement where the rack is moved linearly as the switch 252 is moved and the pinion rotates the ratchet wheel as a result.

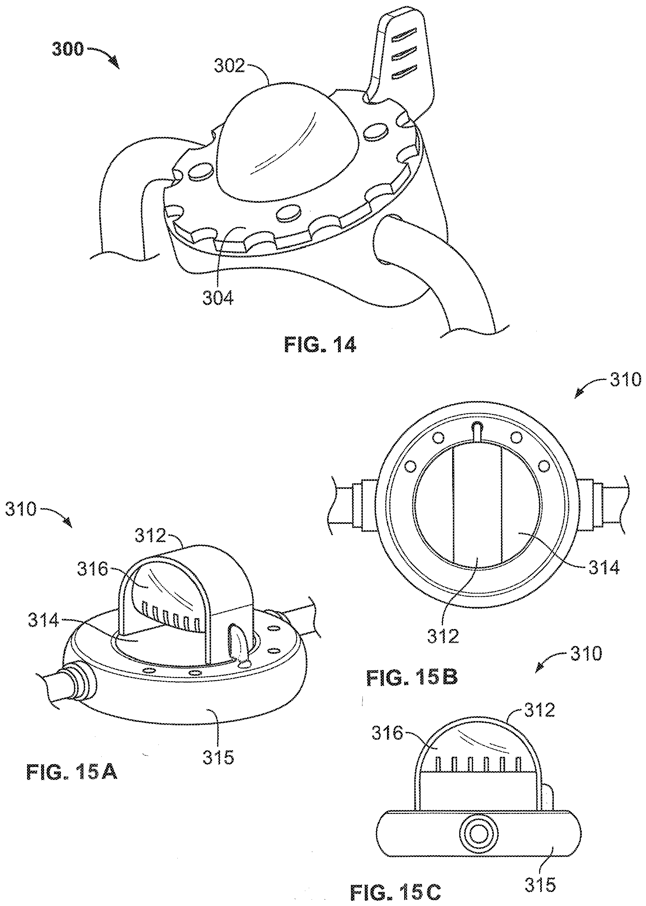

With reference to Fig. 14, an integrated controller and pump device, indicated in general at 300, may feature an integrated push bulb 302 as the pump mechanism. This embodiment may include a valve selector wheel 304 which is connected to a ratchet wheel of a valve mechanism of the type illustrated in Figs. 4-9D. The integrated push bulb defines the pumping chamber for the device that serves as the pumping chamber 76 of Fig. 8.

An integrated controller and pump device that is similar to the embodiment of Fig. 14 is indicated in general at 310 in Figs. 15A-15C. In this embodiment, a U-shaped handle 312 is connected to valve selector wheel 314, which is mounted in the base 315 of the device. The handle 312 facilitates configuring the device via a valve mechanism of the type illustrated in Figs. 4-9D, which is positioned within the base 315. A push bulb 316 is integrated into the handle 312 and is squeezed by a user to provide the pumping action of the device. More specifically, the integrated push bulb 316 may communicate with a pumping chamber positioned within the base 315 of the device, such as pumping chamber 76 of Fig. 8, via tubing positioned within the handle 312 of the device.

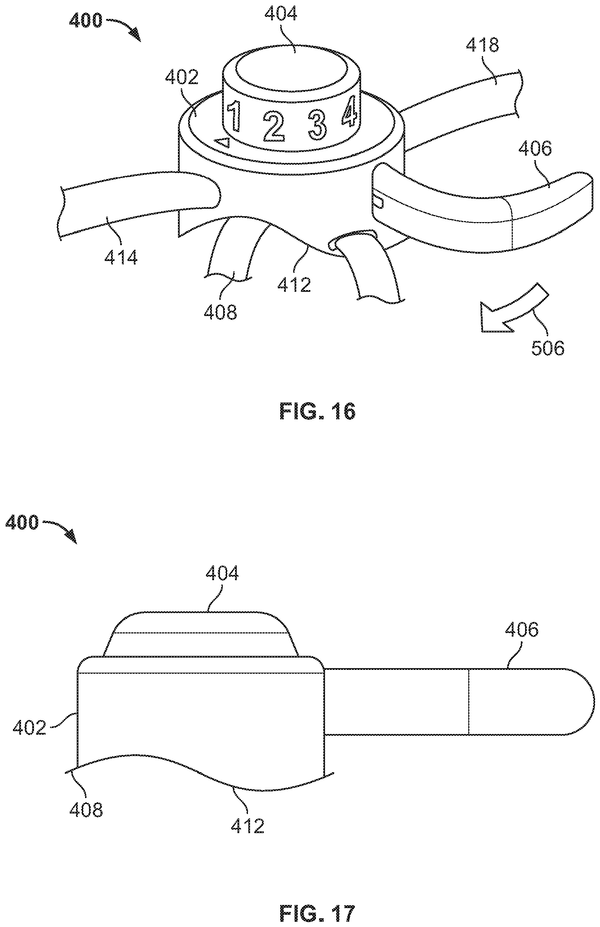

An integrated controller and pump device that uses a rotary pump mechanism is indicated in general at 400 in Figs. 16 and 17. The device includes a housing 402 upon which a rotating valve selector wheel 404 is positioned. In addition, a pumping handle or lever 406 is pivotally attached at its proximal end within the housing for actuating the pump mechanism, as will be explained in greater detail below.

A strap 408 is attached to ends of the housing 402, and the housing preferably features an arcuate bottom surface 412 so that the device may be conveniently mounted on the leg of a user.

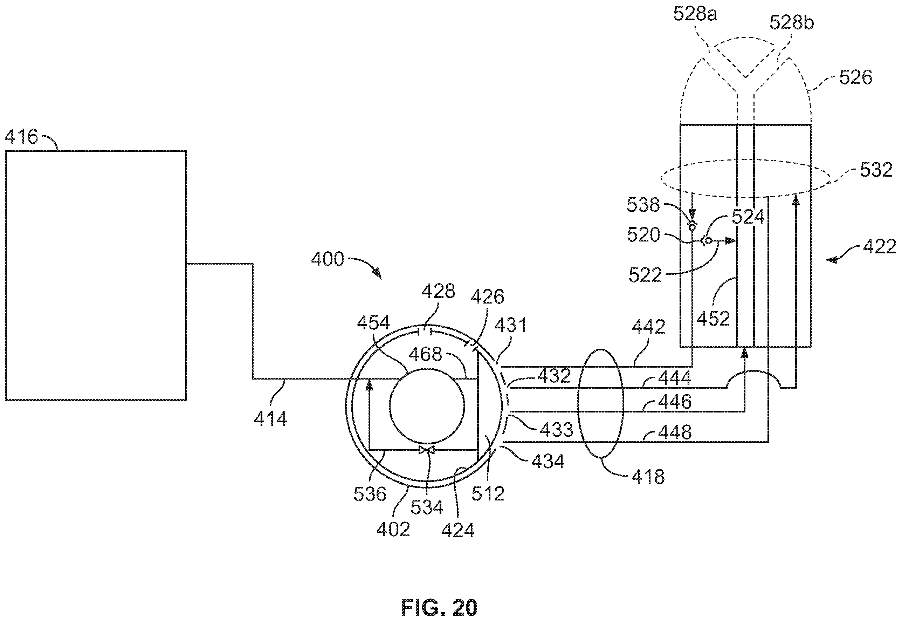

With reference to Fig. 20, inlet tubing 414 connects the pumping mechanism of the device 400 to an irrigant or lavage liquid reservoir illustrated at 416. In addition, outlet tubing 418, which is composed of multiple conduits, connects the pumping mechanism of the device 402 to a rectal catheter 422.

As illustrated in Fig. 20, the device 400 features a valve mechanism that includes a valve cylinder 424 that is pivotally positioned within the housing 402 and connected to the valve selector wheel 404 (Figs. 16 and 17). The valve cylinder 424 is provided with valve apertures 426 and 428, while the housing 402 is provided with outlet ports 431, 432, 433 and 434.

Rotating the valve cylinder 424, via manipulation of the valve selector wheel (404 of Figs. 16 and 17) of the device, selectively places the valve apertures in alignment with or over the outlet ports 431, 432, 433 and 434 so that the integrated controller and pump device may be configured for performing different steps or stages in a transanal irrigation procedure.

When the user rotates the valve selector wheel of the device into the Prime position (which may be indicated, for example, as position "1" on the wheel, as illustrated in Fig. 16) the valve cylinder 424 (Fig. 2) rotates and valve aperture 426 is positioned over outlet port 431. As a result, the device is configured to prime the flushing passage of the catheter. The remaining outlet ports (432-434 of Fig. 20) are covered by the wall of the cylinder 424.

With reference to Fig. 20, the outlet tubing 418 includes conduits 442, 444, 446 and 448. Conduit 442 is connected to outlet port 431 and, as explained in greater detail below, directs the flushing liquid to the flushing passage 452 of the catheter 422.

Continuing with Fig. 20, water, or any other liquid irrigant, is next pumped by the user from reservoir 416 to the catheter flushing passage 452 via actuation of the pumping handle (406 of Figs. 16 and 17) of the rotary pump mechanism 454 of the integrated controller and pump device.

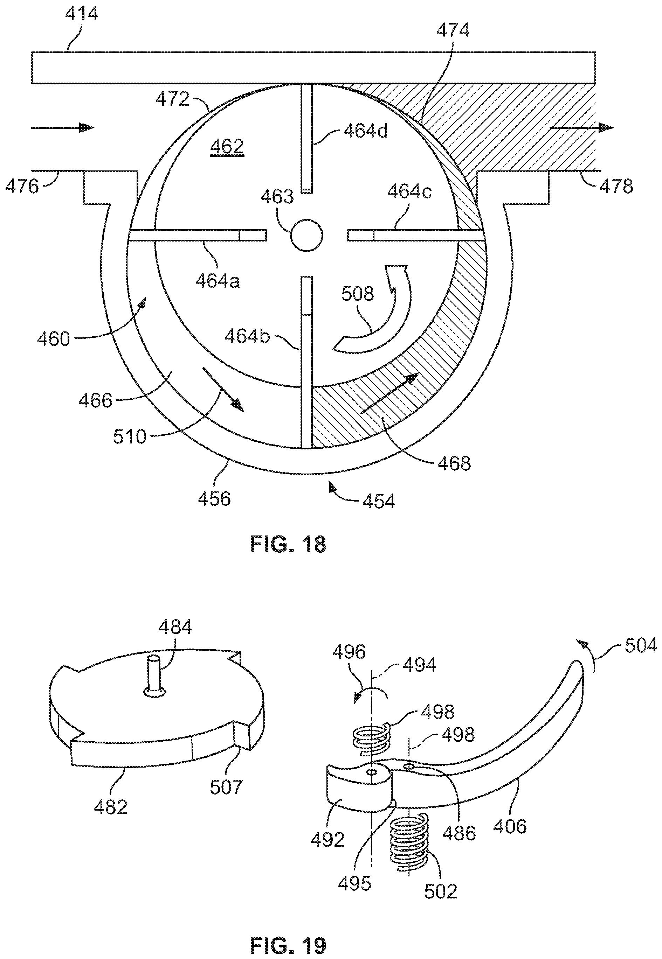

In one embodiment, the rotary pump mechanism 454 takes the form illustrated in Fig. 18. More specifically, the pump mechanism features a pump housing 456 within which is positioned a rotor, indicated in general at 460. The rotor includes a pump wheel 462 that is rotationally mounted within the housing via rotational axis 463. The pump wheel includes four slots within which are positioned vanes 464a, 464b, 464c and 464d. The vanes are configured to slide in a radial direction and are urged away from the rotational axis 463 via coil compression springs (not shown) positioned within the slots of the pump wheel 462. As a result, as illustrated in Fig. 18, the distal ends of the vanes 464a-464d traverse the inner surface of the wall of the pump housing 456 as the rotor is rotated about axis 463 to form liquid pumping chambers, such as 466 and 468. Tracks formed within the inner surfaces of the top and bottom of the pump housing 456 or a curved grid or grating structure or wall with openings positioned along sections 472 and 474 properly retain and position the radial positions of the vanes as they pass over the inlet port 476 and outlet port 478 of the pump housing 456.

While four vanes 464a-464d are illustrated, the rotor 460 may include a different number of vanes.

With reference to Fig. 19, a ratchet wheel 482 is mounted below pump mechanism 454 and is positioned within the device housing 402 (Figs. 16, 17 and 20) of the device 400. A central, upwardly-extending shaft 484 (Fig. 19) connects the ratchet wheel to the rotational axis 463 of the rotor 460 so that the rotor turns when the ratchet wheel turns.

With continued reference to Fig. 19, the proximal end portion of the pumping handle 406 is pivotally mounted within the housing 402 of the device via a pin or the like passing through opening 486. As a result the handle 406 pivots about axis 488 as it is actuated. A pawl 492 is also attached to the proximal end portion of the pumping handle 406 so as to pivot about axis 494. A stop 495 formed on the pawl 492 (which may also or alternatively be formed on the proximal end portion of handle 406) limits movement of the pawl in the direction indicated by arrow 496 to the position illustrated in Fig. 19. A torsion spring 498 urges the pawl into this position. A second torsion spring 502 urges the handle 406 in the direction of arrow 504 until it engages a forward stopping surface positioned on or within the housing 402.

As the pumping handle 406 is pivoted in the direction of arrow 506 in Fig. 16, the tip of pawl 492 (Fig. 19) engages the teeth 507 of ratchet wheel 482 (Fig. 19) so that, with reference to Fig. 18, the rotor spins counter clockwise (as illustrated by arrow 508). As a result, as illustrated by arrows 510, liquid is pumped through the pump mechanism 454 by the vanes 464a-464d. The pumped liquid exits the pump mechanism through pump housing outlet 478 and, with reference to Fig. 20, travels to valve chamber 512. Ratchet wheel 482 may include a number of teeth 507 that differs from the number illustrated in Fig. 19.

When the pump handle 406 is released, torsion spring 502 (Fig. 19) propels it back to the forward home or starting position (i.e. in the direction of arrow 504 of Fig. 19) so that the pumping stroke may be easily repeated. The spring-loaded pivoting attachment of the pawl 492 to the handle permits the teeth of the rotating ratchet wheel 482 to pass as the handle returns to the home or starting position.

With reference to Fig. 20, due to the alignment of valve aperture 426 with outlet port 431, the pumped liquid is pushed out of the valve chamber 512 through the outlet port 431 and conduit 442.

With reference to Fig. 20, the liquid traveling through conduit 442 is directed to the catheter 422 where it encounters junction 520. Branch 522 features a priming check valve 524 that permits the liquid to flow to the catheter flushing passage 452. The catheter flushing passage 452 leads to the catheter tip, illustrated in phantom 526, which includes eyelets 528a and 528b. The user knows that the catheter has been primed when he or she observes the flushing liquid exiting the eyelets 528a and 528b.

The configuration of the catheter may vary from what is illustrated, and it may contain a different number of eyelets.

After the unit is primed, the user will insert the catheter into their rectum, per clinical instructions. The user next rotates the valve selector wheel 404 (Figs. 16 and 17) into the Retention Balloon Inflation position (which may be indicated, for example, as position "2" on the wheel). This causes the cylinder 424 (Fig. 20) to rotate into the position where outlet port 432 is aligned with valve aperture 426. The remaining outlet ports are covered by the cylinder wall.

As illustrated in Fig. 20, outlet port 432 communicates with conduit 444, which leads to the catheter retention balloon 532. The user then actuates the pumping handle 406 (Figs. 16 and 17) which causes liquid to be pumped, in the manner described above with respect to Fig. 18, to the retention balloon 532 so as to inflate it.

The next step in the TAI process is to deliver water into the distal rectum. The user rotates the valve selector wheel 404 (Figs. 16 and 17) to the Flushing position (which may be indicated, for example, as position "3" on the wheel). This causes the cylinder 424 (Fig. 20) to align the valve aperture 426 with the outlet port 433 (with the remaining outlet ports closed by the wall of cylinder 424). As illustrated in Fig. 20, outlet port 433 communicates with conduit 446, which leads to the flushing passage 452 of the catheter 422. The user again manipulates the pumping handle (406 of Figs. 16 and 17) of the device to deliver flushing liquid into the rectum of the user through the flushing passage 452 (Fig. 20) and eyelets 528a and 528b of the catheter 422.

Conduit 446 preferably features a diameter that is larger than the diameter of conduit 444 so that appropriate amounts of liquid are delivered to the rectum and retention balloon during actuation of the pumping handle of the device.

After the correct volume of water has been delivered into the rectum, the user must deflate the retention balloon so that they can remove the catheter from their rectum. Hence, the user rotates the valve selector wheel 404 (Figs. 16 and 17) to the Retention Balloon Deflation position (which may be indicated, for example, as position "4" on the wheel). This causes the cylinder 424 of Fig. 20 to rotate into the position where the valve apertures 426 and 428 are aligned with outlet ports 434 and 431, respectively. In addition, selecting position 4 on the valve selector wheel 404 opens return valve 534 (which is closed when the valve selector wheel is in any of positions 1-3). Alternatively, a separate selector button or switch may be used for opening and closing return valve 534. As a result, with reference to Fig. 20, the water inside the retention balloon 532 is forced by the resiliency of the balloon to returns to the water reservoir 416 via the conduit 448, valve chamber 512, pump mechanism bypass line 536, and tubing 414, thus enabling the balloon to deflate.

A balloon deflation check valve 538 (Fig. 20) also permits liquid from the retention balloon 532 to travel through conduit 442 and port 431 to expedite deflation of the balloon. This feature is optional and the omission of the feature would permit the balloon deflation check valve 538, and the associated branch between the balloon and the junction 520, to be eliminated so that liquid from the deflating balloon only travels through conduit 448. In such an embodiment, only outlet port 434 would be uncovered in Fig. 20.

It should be noted from the above that the lumens of conduits 442, 444, 446 and 448 are never placed in communication with each other during a TAI procedure, i.e. there is no fluid communication between the lumens.

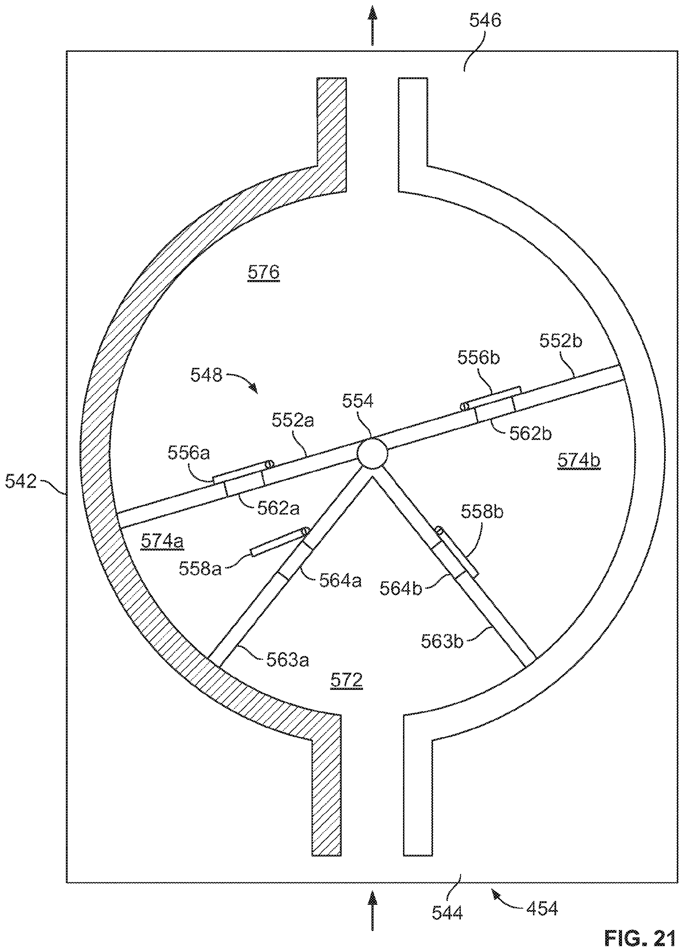

An alternative embodiment of the pump mechanism 454 of Fig. 20 is illustrated in Fig. 21. In this mechanism, the cylindrical pump housing 542 is provided with an inlet port 544, which is connected to line 414 (Fig. 20) and an outlet port 546, which leads to the valve chamber 512 (Fig. 20). The mechanism of Fig. 21 features a rotor, indicated in general at 548, which includes vanes 552a and 552b. Vanes 552a and 552b are provided with valve members 556a and 556b, respectively. Valve members 556a and 556b each pivot so that they may move between a closed position, where corresponding openings 562a and 562b are covered, and an open position where the openings are uncovered.

As illustrated in Fig. 21, a pair of walls 563a and 563b are secured in a fixed fashion within the housing 542 to form a V-shaped structure. The fixed walls feature valve members 558a and 558b that each pivot so that they may move between a closed position, where corresponding openings 564a and 564b are covered, and an open position where the openings are uncovered.

The valve members of Fig. 21 may optionally feature torsion springs that urge them into their closed positions.

The rotor vanes 552a and 552b and walls 563a and 563b divide the interior of the pump housing 542 into inlet chamber 572, transfer chambers 574a and 574b and outlet chamber 576.

The rotor 548 is positioned within the housing 542 so as to rotate about rotational axis 554. In contrast to the embodiment of Figs. 18 and 19, the rotational axis 554 is directly connected to the proximal end of the pumping handle 406 of Figs. 16 and 17. As a result, the rotor 548 of Fig. 21 is pivoted clockwise when the pumping handle 406 of Fig. 16 is turned in the direction of arrow 506. Conversely, when the pumping handle 406 is moved in the opposite direction (in the direction opposite to arrow 506 of Fig. 16), the rotor 548 of Fig. 21 pivots counter clockwise.

During pumping, the pumping handle 406 of Fig. 16 is moved in the direction of arrow 506 and in the opposite direction in an alternating fashion. As a result, the rotor 542, and thus vanes 552a and 552b, of Fig. 21 pivot in a clockwise direction and a counter clockwise direction in an alternating fashion.

When the rotor pivots clockwise, valve member 556a and 558b are closed and valve members 558a and 556b open. As a result, liquid from the inlet chamber 572 is drawn into transfer chamber 574a through opening 564a and liquid is forced out of transfer chamber 574b through opening 562b into outlet chamber 576. In addition, liquid from outlet chamber 576 is forced or pumped out of outlet port 546.

When the rotor pivots counter clockwise, valve members 556b and 558a are closed and valve members 556a and 558b are open. As a result, liquid from the inlet chamber 572 is drawn into transfer chamber 574b through opening 564b and liquid is forced out of transfer chamber 574a through opening 562a into outlet chamber 576. In addition, liquid from outlet chamber 576 is forced or pumped out of outlet port 546.

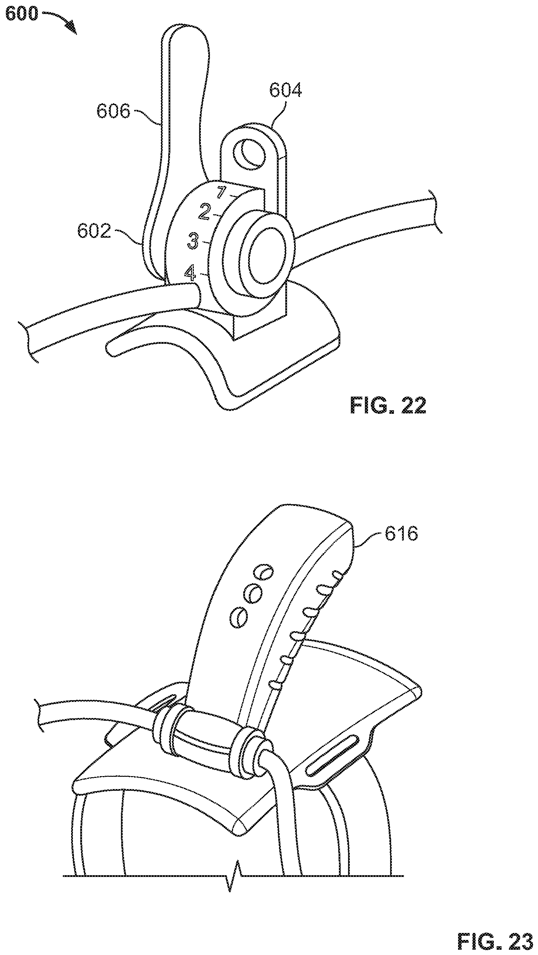

An alternative embodiment of an integrated controller and pump device that uses a rotary pump mechanism is indicated in general at 600 in Fig. 22. The device, which operates in the same fashion as described above with reference to Figs. 16-21, includes a housing 602 upon which a valve selector lever 604 is positioned. In addition, a pumping handle or lever 606 is pivotally attached at its proximal end within the housing for actuating the pump mechanism.

An alternative embodiment of the pumping handle is indicated at 616 in Fig. 23.

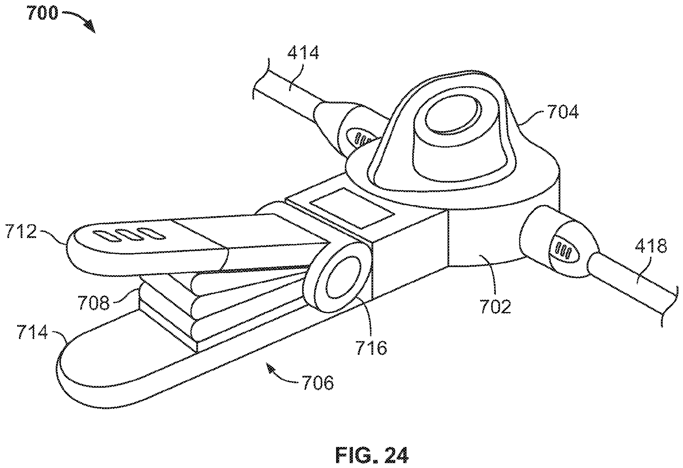

An embodiment of an integrated controller and pump device that uses a detachable bellows pump mechanism is indicated in general at 700 in Fig. 24. The device includes a housing 702 upon which a valve selector wheel 704 is positioned. In addition, a pumping bellows assembly, indicated in general at 706, is removably attached to the housing 702 and serves as the pump mechanism for the device.

The pumping bellows assembly 706 replaces the rotary pumping mechanism 454 of Fig. 20, while the valve selector wheel 704 of Fig. 24 is connected to the valve cylinder 424 of Fig. 20. The valve selector wheel 704 of Fig. 24 therefore moves the valve cylinder 424 of Fig. 20 so that valve configuration for the device for a TAI procedure is accomplished in the same manner as described above with reference to Fig. 20.

The pumping bellows assembly 706 of Fig. 24 includes a bellows 708 that contains an interior pumping chamber that is in liquid communication with the valve chamber 512 of Fig 20. The bellows 708 is mounted within a pumping bellows frame that features top and bottom plates 712 and 714 that are joined by a hinge arrangement 716. The bellows 708 may be constructed from rubber or any other liquid impermeable material that is at least semi-flexible. A spring (not shown) is positioned between and engages the top plate 712 and bottom plate 714 so as to urge the plates into the positions shown in Fig. 24. The spring may optionally be incorporated into the hinge arrangement 716. The top and bottom surfaces of the bellows 708 are attached to the plates 712 and 714 such as by adhesive, and thus the bellows is urged into the expanded configuration illustrated in Fig. 20.

Liquid is pumped out of the pumping chamber of the bellows through one or more outlet check valves by moving the handles of the top and bottom plates 712 and 714 towards each other. Liquid is drawn into the bellows pumping chamber through inlet check valves when the handles of the top and bottom plates move away from each other.

While the preferred embodiments of the disclosure have been shown and described, it will be apparent to those skilled in the art that changes and modifications may be made in line with the appended claims.

## Claims

1. A device for irrigating a body cavity using irrigation liquid from a reservoir and a catheter having a flushing port and a retention balloon comprising:
a. a device housing (42);
b. a pump mechanism (72,76) positioned within the device housing;
c. a valve mechanism positioned within the device housing and including:
i) a valve housing (74) having an inlet port (86) in fluid communication with the pump mechanism and configured to be placed in fluid communication with the reservoir (62), said valve housing also having a plurality of outlet ports (91, 92, 93, 94), at least one of said plurality of outlet ports configured to be placed in fluid communication with the flushing port (144) of the catheter (66) and at least one of said plurality of outlet ports configured to be placed in fluid communication with the retention balloon (172) of the catheter;
ii) a valve member (106) rotatably positioned within the valve housing, said valve member including a valve aperture (120) in fluid communication with the inlet port that may be selectively and individually aligned with each of said plurality of outlet ports;
d. and **characterized in that** said pump mechanism is configured to receive liquid from the valve housing inlet.

2. The device of claim 1 wherein the pump mechanism is positioned within the valve housing.

3. The device of claim 2 wherein the valve housing is cylindrical, the valve member is a disk (106) and the pump mechanism includes a piston (72) positioned within the valve housing and wherein the inlet port and plurality of outlet ports are configured so that liquid is drawn into the cylindrical housing through the inlet port when the piston is moved in a first direction and liquid is forced out of the cylindrical housing when the piston is moved in a second direction.

4. The device of claim 3 further comprising a ratchet wheel (102) connected to the disk so that when the ratchet wheel is turned, the disk is turned, a valve selector (44) positioned on the device housing and connected to the ratchet wheel so that when the valve selector is moved, the ratchet wheel is turned, and a pawl (108) configured to engage teeth (116) of the ratchet wheel.

5. The device of any one of claims 3-4 further comprising a rod (52) attached to the piston and a pumping handle (46) having a proximal end pivotally attached to the device housing and a distal end attached to the rod.

6. The device of any one of claims 3-5 wherein the piston has a valve opening selectively covered by a valve member (73).

7. The device of claim 2 wherein the pump mechanism includes:
d. a pump housing (456) positioned within the valve housing;
e. a rotor (460) rotatably mounted within the pump housing and including a plurality of vanes (464a, 464b, 464c, 464d); and
f. a handle (406) attached to the rotor and configured to turn the rotor.

8. The device of claim 7 wherein the rotor includes a pump wheel (462) having a plurality of slots within which the vanes are slidably received so as to move between retracted positions and extended positions and further comprising a plurality of compression springs positioned within the slots and configured to urge the vanes into the extended positions.

9. The device of claim 7 wherein the pump housing is cylindrical and the plurality of vanes each include a valve opening (562a, 562b, 564a, 564b) and a valve member (556a, 556b, 558a, 558b) configured to selectively cover the valve opening.

10. The device of claim 9 further comprising first and second fixed walls positioned within the plump housing in a V-shaped configuration so that an inlet chamber is defined therebetween, a first transfer chamber is defined between the first fixed wall and a first one of the plurality of vanes, a second transfer chamber is defined between the second fixed wall and a second one of the plurality of vanes and an outlet chamber is defined between the first and second ones of the plurality of vanes, wherein said first and second fixed walls each has a valve opening and a valve member configured to selectively cover the valve opening.

11. The device of any of the preceding claims wherein two of said plurality of outlet ports are configured to be placed in fluid communication with the retention balloon of the catheter for deflation of the retention balloon and wherein the disk valve member includes two valve apertures configured to be simultaneously aligned with the two of said plurality of outlet ports that are configured to be placed in fluid communication with the retention balloon for deflation of the retention balloon.

12. The device of any one of claims 7-10 further comprising a ratchet wheel (482) attached to the rotor and wherein the handle (406) is pivotally mounted within the device housing and configured to turn the ratchet wheel.

13. The device of claim 1 wherein the pump mechanism includes a bellows assembly (706).

14. The device of claim 13 wherein the bellows assembly is removably attached to the device housing.

15. The device of claim 1 wherein the pump mechanism includes a push bulb 302).

## Patentansprüche

1. Vorrichtung zum Spülen eines Körperhohlraums unter Verwendung von Spülflüssigkeit aus einem Behälter und einem Katheter mit einer Spülöffnung und einem Halteballon, umfassend:
a. ein Vorrichtungsgehäuse (42);
b. einen Pumpmechanismus (72, 76), der innerhalb des Vorrichtungsgehäuses positioniert ist;
c. einen Ventilmechanismus, der innerhalb des Vorrichtungsgehäuses positioniert ist und Folgendes beinhaltet:
i) ein Ventilgehäuse (74), das eine Einlassöffnung (86) in Fluidverbindung mit dem Pumpenmechanismus aufweist, die dazu konfiguriert ist, mit dem Behälter (62) in Fluidverbindung gebracht zu werden, wobei das Ventilgehäuse außerdem eine Vielzahl von Auslassöffnungen (91, 92, 93, 94) aufweist, wobei mindestens eine der Vielzahl von Auslassöffnungen dazu konfiguriert ist, mit der Spülöffnung (144) des Katheters (66) in Fluidverbindung gebracht zu werden, und mindestens eine der Vielzahl von Auslassöffnungen dazu konfiguriert ist, mit dem Halteballon (172) des Katheters in Fluidverbindung gebracht zu werden;
ii) ein Ventilelement (106), das drehbar innerhalb des Ventilgehäuses positioniert ist, wobei das Ventilelement eine Ventilöffnung (120) in Fluidverbindung mit der Einlassöffnung aufweist, die selektiv und individuell an jeder der Vielzahl von Auslassöffnungen ausgerichtet werden kann;
d. und **dadurch gekennzeichnet, dass** der Pumpmechanismus dazu konfiguriert ist, Flüssigkeit aus dem Ventilgehäuseeinlass aufzunehmen.

2. Vorrichtung nach Anspruch 1, wobei der Pumpmechanismus innerhalb des Ventilgehäuses positioniert ist.

3. Vorrichtung nach Anspruch 2, wobei das Ventilgehäuse zylindrisch ist, das Ventilelement eine Scheibe (106) ist und der Pumpmechanismus einen innerhalb des Ventilgehäuses positionierten Kolben (72) beinhaltet und wobei die Einlassöffnung und die Vielzahl von Auslassöffnungen so konfiguriert sind, dass Flüssigkeit durch die Einlassöffnung in das zylindrische Gehäuse gesaugt wird, wenn der Kolben in eine erste Richtung bewegt wird, und Flüssigkeit aus dem zylindrischen Gehäuse getrieben wird, wenn der Kolben in eine zweite Richtung bewegt wird.

4. Vorrichtung nach Anspruch 3, ferner umfassend ein Klinkenrad (102), das mit der Scheibe verbunden ist, so dass, wenn das Klinkenrad gedreht wird, die Scheibe gedreht wird, einen Ventilwähler (44), der an dem Vorrichtungsgehäuse positioniert und mit dem Klinkenrad verbunden ist, so dass, wenn der Ventilwähler bewegt wird, das Klinkenrad gedreht wird, und eine Sperrklinke (108), die dazu konfiguriert ist, mit Zähnen (116) des Klinkenrads in Eingriff zu stehen.

5. Vorrichtung nach einem der Ansprüche 3-4, ferner umfassend eine an dem Kolben angebrachte Stange (52) und einen Pumpgriff (46), der ein schwenkbar an dem Vorrichtungsgehäuse angebrachtes proximales Ende und ein an der Stange angebrachtes distales Ende aufweist.

6. Vorrichtung nach einem der Ansprüche 3-5, wobei der Kolben eine Ventilöffnung aufweist, die selektiv durch ein Ventilelement (73) abgedeckt wird.

7. Vorrichtung nach Anspruch 2, wobei der Pumpmechanismus Folgendes beinhaltet:
d. ein Pumpengehäuse (456), das innerhalb des Ventilgehäuses positioniert ist;
e. einen Rotor (460), der drehbar innerhalb des Pumpengehäuses montiert ist und eine Vielzahl von Schaufeln (464a, 464b, 464c, 464d) beinhaltet; und
f. einen Griff (406), der an dem Rotor angebracht und dazu konfiguriert ist, den Rotor zu drehen.

8. Vorrichtung nach Anspruch 7, wobei der Rotor ein Pumpenrad (462) beinhaltet, das eine Vielzahl von Schlitzen aufweist, in denen die Schaufeln verschiebbar aufgenommen sind, so dass sie sich zwischen eingefahrenen Positionen und ausgefahrenen Positionen bewegen, und ferner umfassend eine Vielzahl von in den Schlitzen positionierten Druckfedern, die dazu konfiguriert ist, die Schaufeln in die ausgefahrenen Positionen zu drängen.

9. Vorrichtung nach Anspruch 7, wobei das Pumpengehäuse zylindrisch ist und die Vielzahl von Schaufeln jeweils eine Ventilöffnung (562a, 562b, 564a, 564b) und ein Ventilelement (556a, 556b, 558a, 558b) beinhaltet, das dazu konfiguriert ist, die Ventilöffnung selektiv abzudecken.

10. Vorrichtung nach Anspruch 9, ferner umfassend eine erste und eine zweite feststehende Wand, die innerhalb des plumpen Gehäuses in einer V-förmigen Konfiguration angeordnet sind, so dass eine Einlasskammer zwischen ihnen definiert ist, wobei eine erste Übertragungskammer zwischen der ersten feststehenden Wand und einer ersten einen der Vielzahl von Schaufeln definiert ist, eine zweite Übertragungskammer zwischen der zweiten feststehenden Wand und einer zweiten einen der Vielzahl von Schaufeln definiert ist und eine Auslasskammer zwischen den ersten und zweiten einen der Vielzahl von Schaufeln definiert ist, wobei die erste und die zweite feststehende Wand jeweils eine Ventilöffnung und ein Ventilelement aufweisen, das dazu konfiguriert ist, die Ventilöffnung selektiv abzudecken.

11. Vorrichtung nach einem der vorangehenden Ansprüche, wobei zwei der Vielzahl von Auslassöffnungen dazu konfiguriert sind, zum Entleeren des Halteballons in Fluidverbindung mit dem Halteballon des Katheters gebracht zu werden, und wobei das Scheibenventilelement zwei Ventilöffnungen beinhaltet, die dazu konfiguriert sind, gleichzeitig an den zwei der Vielzahl von Auslassöffnungen ausgerichtet zu sein, die dazu konfiguriert sind, zum Entleeren des Halteballons in Fluidverbindung mit dem Halteballon des Katheters gebracht zu werden.

12. Vorrichtung nach einem der Ansprüche 7-10, ferner umfassend ein Klinkenrad (482), das an dem Rotor angebracht ist, und wobei der Griff (406) schwenkbar innerhalb des Vorrichtungsgehäuses montiert und dazu konfiguriert ist, das Klinkenrad zu drehen.

13. Vorrichtung nach Anspruch 1, wobei der Pumpmechanismus eine Faltenbalgbaugruppe (706) beinhaltet.

14. Vorrichtung nach Anspruch 13, wobei die Faltenbalgbaugruppe abnehmbar an dem Vorrichtungsgehäuse angebracht ist.

15. Vorrichtung nach Anspruch 1, wobei der Pumpmechanismus einen Druckkolben 302) umfasst.

## Revendications

1. Dispositif d'irrigation d'une cavité corporelle à l'aide d'un liquide d'irrigation provenant d'un réservoir et d'un cathéter ayant un orifice de rinçage et un ballonnet de rétention comprenant :
a. un boîtier de dispositif (42) ;
b. un mécanisme de pompe (72, 76) positionné à l'intérieur du boîtier de dispositif ;
c. un mécanisme de soupape positionné à l'intérieur du boîtier de dispositif et comportant :
i) un boîtier de soupape (74) ayant un orifice d'entrée (86) en communication fluidique avec le mécanisme de pompe et configuré pour être placé en communication fluidique avec le réservoir (62), ledit boîtier de soupape ayant également une pluralité d'orifices de sortie (91, 92, 93, 94), l'un au moins de ladite pluralité d'orifices de sortie étant configuré pour être placé en communication fluidique avec l'orifice de rinçage (144) du cathéter (66) et l'un au moins de ladite pluralité d'orifices de sortie étant configuré pour être placé en communication fluidique avec le ballonnet de rétention (172) du cathéter ;
ii) un élément de soupape (106) positionné de manière rotative à l'intérieur du boîtier de soupape, ledit élément de soupape comportant une ouverture de soupape (120) en communication fluidique avec l'orifice d'entrée qui peut être alignée sélectivement et individuellement avec chacun de ladite pluralité d'orifices de sortie ;
d. et **caractérisé en ce que** ledit mécanisme de pompe est configuré pour recevoir du liquide provenant de l'entrée du boîtier de soupape.

2. Dispositif selon la revendication 1, dans lequel le mécanisme de pompe est positionné à l'intérieur du boîtier de soupape.

3. Dispositif selon la revendication 2, dans lequel le boîtier de soupape est cylindrique, l'élément de soupape est un disque (106) et le mécanisme de pompe comporte un piston (72) positionné à l'intérieur du boîtier de soupape et dans lequel l'orifice d'entrée et la pluralité d'orifices de sortie sont configurés de sorte que le liquide est aspiré dans le boîtier cylindrique à travers l'orifice d'entrée lorsque le piston est déplacé dans une première direction et que le liquide est expulsé hors du boîtier cylindrique lorsque le piston est déplacé dans une seconde direction.

4. Dispositif selon la revendication 3 comprenant en outre une roue à rochet (102) reliée au disque de sorte que lorsque la roue à rochet est tournée, le disque est tourné, un sélecteur de soupape (44) positionné sur le boîtier du dispositif et relié à la roue à rochet de sorte que lorsque le sélecteur de soupape est déplacé, la roue à rochet est tournée, et un cliquet (108) configuré pour engager les dents (116) de la roue à rochet.

5. Dispositif selon l'une quelconque des revendications 3 et 4 comprenant en outre une tige (52) fixée au piston et une poignée de pompage (46) ayant une extrémité proximale fixée de manière pivotante au boîtier de dispositif et une extrémité distale fixée à la tige.

6. Dispositif selon l'une quelconque des revendications 3 à 5, dans lequel le piston a une ouverture de soupape recouverte sélectivement par un élément de soupape (73).

7. Dispositif selon la revendication 2, dans lequel le mécanisme de pompe comporte :
d. un boîtier de pompe (456) positionné à l'intérieur du boîtier de soupape ;
e. un rotor (460) monté de manière rotative à l'intérieur du boîtier de pompe et comportant une pluralité d'aubes (464a, 464b, 464c, 464d) ; et
f. une poignée (406) fixée au rotor et configurée pour faire tourner le rotor.

8. Dispositif selon la revendication 7, dans lequel le rotor comporte une roue de pompe (462) ayant une pluralité de fentes à l'intérieur desquelles les aubes sont reçues de manière coulissante de manière à se déplacer entre des positions rétractées et des positions étendues et comprenant en outre une pluralité de ressorts de compression positionnés à l'intérieur des fentes et configurés pour pousser les aubes dans les positions étendues.

9. Dispositif selon la revendication 7, dans lequel le boîtier de pompe est cylindrique et la pluralité d'aubes comportent chacune une ouverture de soupape (562a, 562b, 564a, 564b) et un élément de soupape (556a, 556b, 558a, 558b) configuré pour couvrir sélectivement l'ouverture de soupape.

10. Dispositif selon la revendication 9, comprenant en outre des première et seconde parois fixes positionnées à l'intérieur du boîtier de pompe dans une configuration en forme de V de sorte qu'une chambre d'entrée est définie entre elles, une première chambre de transfert est définie entre la première paroi fixe et une première de la pluralité d'aubes, une seconde chambre de transfert est définie entre la seconde paroi fixe et une seconde de la pluralité d'aubes et une chambre de sortie est définie entre les première et seconde de la pluralité d'aubes, dans lequel lesdites première et secondes parois fixes ont chacune une ouverture de soupape et un élément de soupape configuré pour couvrir sélectivement l'ouverture de soupape.

11. Dispositif selon l'une quelconque des revendications précédentes, dans lequel deux de ladite pluralité d'orifices de sortie sont configurés pour être placés en communication fluidique avec le ballonnet de rétention du cathéter pour le dégonflage du ballonnet de rétention et dans lequel l'élément de soupape à disque comporte deux ouvertures de soupape configurées pour être alignées simultanément avec les deux de ladite pluralité d'orifices de sortie qui sont configurés pour être placés en communication fluidique avec le ballonnet de rétention pour le dégonflage du ballonnet de rétention.

12. Dispositif selon l'une quelconque des revendications 7 à 10, comprenant en outre une roue à rochet (482) fixée au rotor et dans lequel la poignée (406) est montée de manière pivotante dans le boîtier de dispositif et configurée pour faire tourner la roue à rochet.

13. Dispositif selon la revendication 1, dans lequel le mécanisme de pompe comporte un ensemble à soufflet (706).

14. Dispositif selon la revendication 13, dans lequel l'ensemble à soufflet est fixé de manière amovible au boîtier de dispositif.

15. Dispositif selon la revendication 1, dans lequel le mécanisme de pompe comporte une poire de poussée (302).
